(19) Europäisches Patentamt
European Patent Office
Office européen des brevets

(11) Publication number: **0 266 032**
**A1**

(12) # EUROPEAN PATENT APPLICATION

(21) Application number: **87307496.7**

(22) Date of filing: **25.08.87**

(51) Int. Cl.⁴: **C12N 15/00**, C12N 9/68, A61K 37/54

The title of the invention has been amended (Guidelines for Examination in the EPO, A-III, 7.3).

(30) Priority: **29.08.86 GB 8620952**
**29.08.86 GB 8620953**

(43) Date of publication of application:
**04.05.88 Bulletin 88/18**

(84) Designated Contracting States:
**BE CH DE FR GB IT LI NL**

(71) Applicant: **BEECHAM GROUP PLC**
**Beecham House Great West Road**
**Brentford Middlesex TW8 9BD(GB)**

(72) Inventor: **Robinson, Jeffery Hugh Beecham Pharmaceuticals**
**Great Burgh Yew Tree Bottom Road**
**Epsom Surrey, KT18 5XQ(GB)**
Inventor: **Browne, Michael Joseph Beecham Pharmaceuticals**
**Great Burgh Yew Tree Bottom Road**
**Epsom Surrey, KT18 5XQ(GB)**

(74) Representative: **Valentine, Jill Barbara et al**
**Beecham Pharmaceuticals Patents & Trade Marks Dept. Great Burgh Yew Tree Bottom Road**
**Epsom Surrey KT18 5XQ(GB)**

(54) **Modified fibrinolytic enzyme.**

(57) A modified fibrinolytically active urokinase-type plasminogen activator (u-PA) which has been modified in the region of the growth factor domain, the catalytic site essential for fibrinolytic activity of which is optionally blocked by a removable blocking group.

EP 0 266 032 A1

## Novel Compounds

The present invention relates to a modified fibrinolytic enzyme, in particular modified urokinase-type plasminogen activator, its preparation, pharmaceutical compositions containing it and its use in the treatment of thrombotic diseases.

The sequence of amino acids making up the enzyme urokinase-type plasminogen activator (u-PA) in its single chain and two chain forms (Verstraete, M. and Collen, D., 1986; Blood, 67, 1529) and the nucleotide sequence for the cDNA which codes for human u-PA (Holmes, W. E. et al, 1985; Bio/technology 3, 923-929) are known. Urokinase-type plasminogen activator is known to have fibrinolytic activity.

It has been shown (Bányai, L. et al, 1983; FEBS Lett., 163, 37)that a part of the u-PA enzyme shows structural homology with human and murine epidermal growth factors. This region from amino acid residues 5 to 49 has been termed the "growth factor domain". The genetic information which codes for the major part of this domain, residues 10 to 44 inclusive, and partially codes for residues 9 and 45, lies on a single exon (Riccio, A. et al, 1985; Nucl. Acids Res. 13, 2759-2771).

Within this region there is a sequence of three amino acids:

```
         - tyr - phe - ser-
position 24     25    26
```

The applicants have now identified modified forms of the u-PA enzyme which retain fibrinolytic activity.

According to the present invention there is provided a fibrinolytically active urokinase-type plasminogen activator which has been modified in the region of the growth factor domain.

Suitable modifications may include removal or deletion of certain amino acid residues, or replacement of one or more amino acid residues with different amino acid residues.

In one preferred aspect, the modification comprises the deletion of all or part of the growth factor domain.

In another preferred aspect, the u-PA is modified in the region from amino acid residues 11 to 45 inclusive, in particular by deletion of that region.

In a further preferred aspect, the u-PA is modified in the region of amino acids 24 to 26, in particular by deletion of that region.

The modified u-PA preferably comprises amino acids 1 to 10 inclusive of native u-PA.

As used herein, the term urokinase-type plasminogen activator (u-PA) denotes a plasminogen activator of the group having the immunological properties defined for u-PA at the XXVIII Meeting of the International Committee on Thrombosis and Haemostasis, Bergamo, Italy, 27 July 1982.

The numbering system for the amino acid and nucleotide sequence of u-PA used herein is that described in Holmes, W. E. et al, 1985 (op. cit.) in which the N-terminal serine residue is numbered 1.

The modified u-PA of the invention may be derivatised, where appropriate, to provide pharmaceutically useful conjugates analogous to known u-PA-containing conjugates, for example:

(a) an enzyme-protein conjugate as disclosed in EP-A-O 155 388, in which the catalytic site on the enzyme which is responsible for fibrinolytic activity is blocked by a human protein attached thereto by way of a reversible linking group;

(b) an enzyme-protein conjugate as disclosed in EP-A-O 152,736, comprising at least one optionally blocked fibrinolytic enzyme linked by way of a site other than the catalytic site responsible for fibrinolytic activity to at least one human protein;

(c) a protein-polymer conjugate as disclosed in EP-A-0183503 comprising a pharmaceutically useful protein linked to at least one water soluble polymer by means of a reversible linking group; or

(d) an enzyme conjugate as disclosed in EP-A-0184363 comprising a plurality of fibrinolytic enzymes linked together through the active centres thereof by means of a removable blocking group.

The modified u-PA of the invention may take the place of u-PA as the enzyme or (human) protein component, as appropriate, of any of the conjugates described above.

The modified u-PA of the invention or conjugate thereof can be further derivatised such that any catalytic site essential for fibrinolytic activity is optionally blocked by a removable blocking group.

The above mentioned derivatives of the modified u-PA may be used in any of the methods and compositions described hereinafter for the modified u-PA itself.

As used herein the expression 'removable blocking group' includes groups which are removable by hydrolysis at a rate such that the pseudo-first order rate constant for hydrolysis is in the range of $10^{-6}$ $sec^{-1}$ to $10^{-2}$ $sec^{-1}$ in isotonic aqueous media at pH 7.4 at 37°C.

Such blocking groups are described in European Patent No.0009879 and include acyl groups such as optionally substituted benzoyl or optionally substituted acryloyl.

Suitable optional substituents for benzoyl blocking groups include halogen, $C_{1-6}$ alkyl, $C_{1-6}$ alkoxy, $C_{1-6}$ alkanoyloxy, $C_{1-6}$ alkanoylamino, amino or p-guanidino.

Suitable optional substituents for acryloyl blocking groups include $C_{1-6}$ alkyl, furyl, phenyl or $C_{1-6}$alkylphenyl.

In a further aspect, the invention provides a process for preparing modified urokinase-type plasminogen activator according to the invention which process comprises expressing DNA encoding said modified urokinase-type plasminogen activator in a recombinant host cell and recovering the modified urokinase-type plasminogen activator product.

The modification of the u-PA can therefore be carried out by conventional genetic engineering techniques in which the cDNA which codes for u-PA is modified and the modified cDNA expressed in a prokaryote or eukaryote host.

The DNA polymer comprising a nucleotide sequence that encodes the modified u-PA also forms part of the invention.

The process of the invention may be performed by conventional recombinant techniques such as described in Maniatis et. al\., Molecular Cloning - A Laboratory Manual; Cold Spring Harbor, 1982.

In particular, the process may comprise the steps of:

i) preparing a replicable expression vector capable, in a host cell, of expressing a DNA polymer comprising a nucleotide sequence that encodes said modified urokinase-type plasminogen activator;

ii) transforming a host cell with said vector;

iii)culturing said transformed host cell under conditions permitting expression of said DNA polymer to produce said modified urokinase-type plasminogen activator; and

iv) recovering said modified urokinase-type plasminogen activator.

The invention also provides a process for preparing the DNA polymer by the condensation of appropriate mono-, di-or oligomeric nucleotide units.

The preparation may be carried out chemically, enzymatically, or by a combination of the two methods, in vitro or in vivo as appropriate. Thus, the DNA polymer may be prepared by the enzymatic ligation of chemically synthesised DNA fragments, by conventional methods such as those described by D. M. Roberts et al in Biochemistry 1985, 24, 5090-5098. The chemical synthesis may be performed generally as described hereinafter for the synthesis of an oligodeoxyribonucleotide. Enzymatic polymerisation of DNA may be carried out in vitro using a DNA polymerase such as DNA polymerase I (Klenow fragment) in an appropriate buffer containing the nucleoside triphosphates dATP, dCTP, dGTP and dTTP as required at a temperature of l0°-37°C, generally in a volume of 100µl or less. Enzymatic ligation of DNA fragments may be carried out using a DNA ligase such as T4 DNA ligase in an appropriate buffer at a temperature of 4°C to ambient, generally in a volume of 50µl or less. DNA polymer which encodes the modified u-PA may be prepared by site-directed mutagenesis of the cDNA which codes for urokinase-type plasminogen activator, by conventional methods such as those described by G. Winter et al in Nature 1982, 299, 756-758 or by Zoller and Smith 1982; Nucl. Acids Res., 10, 6487-6500, or deletion mutagenesis such as described by Chan and Smith in Nucl. Acids Res., 1984, 12, 2407-2419 or by G. Winter et al in Biochem. Soc. Trans., 1984, 12, 224-225. Expression of the modified cDNA can be carried out by conventional methods.

The above described mutagenesis processes involve the use of oligodeoxyribonucleotides which can be designed according to the changes required in the cDNA sequence coding for amino acids 5 to 49 inclusive, such as amino acids 24 to 26 inclusive.

The oligodeoxyribonucleotides which may be used in such processes also form part of the invention.

The invention also provides a process for preparing an oligodeoxyribonucleotide of the invention, which process comprises the deprotection of a second oligodeoxyribonucleotide having the same sequence of bases as said oligodeoxyribonucleotide and in which all free hydroxy and amino groups are protected.

The oligodeoxyribonucleotides used in the process can be prepared by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982),or in other scientific publications, for example M.J. Gait, H.W.D. Matthes, M. Singh, B.S. Sproat, and R.C. Titmas, Nucleic Acids Research, 1982, 10, 6243; B.S. Sproat and W. Bannwarth, Tetrahedron Letters, 1983, 24, 5771; M.D. Matteucci and M.H Caruthers, Tetrahedron

Letters, 1980, 21, 719; M.D. Matteucci and M.H. Caruthers, Journal of the American Chemical Society, 1981, 103, 3185; S.P. Adams et al., Journal of the American Chemical Society,1983, 105, 661; N.D. Sinha, J. Biernat, J. McMannus, and H. Koester, Nucleic Acids Research, 1984, 12, 4539; and H.W.D. Matthes et al., EMBO Journal, 1984, 3, 801.

Solid supports which may be employed include conventional supports known in the art, for example kieselguhr-polydimethylacrylamide, silica, controlled-pore glass, or cellulose paper discs. The base at the 3′-end of the oligodeoxyribonucleotide to be prepared is attached, via a spacer group connected to the 3′-oxygen of the terminal 2′-deoxyribose unit, to the solid support and assembly of the oligodeoxyribonucleotide (in a protected form) is carried out in the 3′→ 5′-direction by cycles of synthesis, the required operations being performed either manually or by an automated instrument.

At the end of the synthesis, the protected oligodeoxyribonucleotide may be cleaved from the solid support either before, after, or at the same time as other deprotection steps that are carried out once the desired sequence of bases has been assembled, as described hereinbelow. Conveniently the oligodeoxyribonucleotide is removed from the solid support by treatment with a basic reagent such as aqueous ammonia at ambient or slightly elevated temperature, or with 1,1,3,3 -tetramethylguanidinium-syn-2-nitrobenzaldoximate or similar reagents in aqueous dioxan at ambient or slightly elevated temperature.

Alternatively, the preparation may be carried out conventionally by phosphotriester chemistry in solution as described, for example, by C.B. Reese, Tetrahedron, 1978, 34, 3143-3179.

The required sequence of nucleotide bases in the oligodeoxyribonucleotide may be built up conventionally, for example as described in the aforementioned publications, by the condensation of appropriate mono-, di-or oligomeric nucleotide units in the presence of a suitable condensing agent such as 1-(mesitylenesulphonyl)-3-nitro-1,2,4-triazole in a solvent such as pyridine at ambient or slightly elevated temperature (when phosphotriester chemistry is employed) or by a condensing agent such as tetrazole in acetonitrile at ambient temperature (when phosphite or phosphoramidite chemistry is used). Optionally a 'capping' step is introduced after each condensation step to inactivate any unreacted starting material containing a free 5′-hydroxy group. Such a capping step may suitably be carried out by an acylating agent such as acetic anhydride in 2,6-lutidine and 4-N,N-dimethylaminopyridine in tetrahydrofuran at ambient temperature.

When phosphite or phosphoramidite chemistry is employed, the phosphorus (III) atom in the phosphite-triester internucleotide linkage created in each condensation step is oxidised, giving the corresponding phosphotriester linkage, before a further condensation step is carried out. Such oxidations may be carried out using a convenient oxidising agent, for example iodine in aqueous tetrahydrofuran in the presence of a base such as lutidine. The oxidation step is conveniently carried out after the optional 'capping' step as hereinbefore described.

During the synthesis of the oligodeoxyribonucleotide, the hydroxy groups in each of the internucleotide phosphodiester bridges may be protected by an aryl group or alkyl group conventionally employed for the purpose, for example 2-or 4-chlorophenyl, methyl, or 2-cyanoethyl, to prevent branching of the molecule. The aryl protecting groups are removed at the end of the synthesis by treatment with an agent conventionally employed for the purpose, for example 1,1,3,3-tetramethyl-guanidinium-syn -2-nitrobenzaldoximate in an aqueous solvent such as aqueous dioxan at ambient temperature. The methyl groups may be removed at the end of the synthesis by treatment with triethylammonium thiophenolate in dioxan at ambient temperature. The 2-cyanoethyl groups may be removed at the end of the synthesis with a basic reagent such as triethylamine or tert-butylamine in pyridine at ambient temperature, or alternatively by treatment with concentrated aqueous ammonia at about 50°C (a step which simultaneously and advantageously removes the protecting groups present on the A, C and G bases as described hereinbelow).

The amino groups present in the A, C and G bases are protected by base-labile protecting groups, such as benzoyl for A and C, and isobutyryl for G. Such groups may be removed by treatment with basic reagents, for example ammonia at ambient or slightly elevated temperature, for example 50°C. The 5′-hydroxy group of the terminal ribose moiety is protected by an acid-labile group such as trityl, 4,4′-dimethoxytrityl or pixyl (9-phenyl-9-xanthyl), which is removed before each condensation step and at the end of synthesis. Such groups may be removed by treatment with an acidic reagent such as di-or trichloroacetic acid in an anhydrous solvent such as dichloromethane or chloroform, at ambient temperature.

The expression of the DNA polymer encoding the modified u-PA in a recombinant host cell may be carried out by means of a replicable expression vector capable, in the host cell, of expressing the DNA polymer. The expression vector is novel and also forms part of the invention.

The replicable expression vector may be prepared in accordance with the invention, by cleaving a vector compatible with the host cell to provide a linear DNA segment having an intact replicon, and combining said linear segment with the DNA polymer encoding the modified u-PA under ligating conditions.

The choice of vector will be determined in part by the host cell, which may be prokaryotic or eukaryotic. Suitable vectors include plasmids, bacteriophages, recombinant viruses and cosmids.

The preparation of the replicable expression vector may be carried out conventionally with appropriate enzymes for restriction, polymerisation and ligation of the DNA, by procedures described in, for example, Maniatis et al cited above. Polymerisation and ligation may be performed as described above for the preparation of the DNA polymer. Digestion with restriction enzymes may be performed in an appropriate buffer at a temperature of 20°-70°C, generally in a volume of 200μl or less with 0.1-10μg DNA.

The recombinant host cell is prepared, in accordance with the invention, by transforming a host cell with a replicable expression vector of the invention under transforming conditions. Suitable transforming conditions are conventional and are described in, for example, Maniatis et al cited above, or "DNA Cloning" Vol. II, D.M. Glover ed., IRL Press Ltd, 1985.

The choice of transforming conditions is determined by the host cell. Thus, a bacterial host such as E. coli may be treated with a solution of $CaCl_2$ (Cohen et al, Proc. Nat. Acad. Sci., 1973, 69, 2110) or with a solution comprising a mixture of RbCl, $MnCl_2$, potassium acetate and glycerol, and then with 3-[N-morpholino]-propane-sulphonic acid, RbCl and glycerol. Mammalian cells in culture may be transformed by calcium co-precipitation of the vector DNA onto the cells. The invention also extends to a host cell transformed with a replicable expression vector of the invention.

Culturing the transformed host cell under conditions permitting expression of the DNA polymer is carried out conventionally, as described in, for example, Maniatis et al and "DNA Cloning" cited above. Thus, preferably the cell is supplied with nutrient and cultured at a temperature below 45°C.

The modified u-PA expression product is recovered by conventional methods according to the host cell. Thus, where the host cell is bacterial, such as E. coli it may be lysed physically, chemically or enzymatically and the protein product isolated from the resulting lysate. Where the host cell is mammalian, the product may generally be isolated from the nutrient medium. The DNA polymer may be assembled into vectors designed for isolation of stable transformed mammalian cell lines expressing the modified u-PA; e.g. bovine papillomavirus vectors (DNA cloning Vol.II D.M. Glover Ed. IRL Press 1985; Kaufman, R.J. et al, Molecular and Cellular Biology 5, 1750-1759, 1985; Pavlakis G.N. and Hamer, D.H., Proceedings of the National Academy of Sciences (USA) 80, 397-401, 1983; Goeddel, D.V. et al., European Patent Application No. 0093619, 1983).

It will be appreciated that, depending upon the host cell, the modified u-PA prepared in accordance with the invention may be glycosylated to varying degrees. The modified u-PA of the invention is understood to include such glycosylated variations.

In one preferred embodiment, a modified urokinase-type plasminogen activator enzyme is prepared by a process which comprises effecting a deletion mutagenesis upon the cDNA which codes for urokinase-type plasminogen activator using an oligodeoxyribonucleotide comprising a nucleotide sequence which is complementary or corresponds to a region of the u-PA cDNA extending 5′ from nucleotide 166 inclusive, said sequence being joined directly to a nucleotide sequence which is complementary or corresponds to a region extending 3′ from nucleotide 272 inclusive of u-PA cDNA and expressing the modified cDNA in a prokaryote or eurokaryote host.

The oligodeoxyribonucleotide employed in the preferred embodiment is suitably at least 16 nucleotides in length, preferably at least 30 nucleotides in length. Suitably it contains at least 8 and preferably at least 15 nucleotides which are complementary to nucleotides on each side of the deletion region.

A preferred oligodeoxyribonucleotide is of sequence (I).

5′d (GCAGGTTTTTGACTTGTTCGATGGAACTTG) 3′ (I)

The oligodeoxyribonucleotide of sequence (I) is novel and as such forms part of the invention.

The 15 bases at the 5′ end of the oligodeoxyribonucleotide (I) are complementary to u-PA cDNA nucleotides 286-272 inclusive and the 15 bases at the 3′ end of the oligodeoxyribonucleotide (I) are complementary to the u-PA cDNA nucleotides 166-152 inclusive.

The oligodeoxyribonucleotide of sequence (I) can be used to delete the portion of cDNA which codes for amino acids 11 to 45 inclusive of u-PA. It is believed that the cDNA obtained following mutagenesis is similar to that of Holmes, W. E. et al, 1985, (Op. cit.) with the following new nucleotide sequence:-

```
5' - CAA GTT CCA TCG AAC AAG TCA AAA ACC TGC 3'
          |                  | |              |
position 152               166 272           286
```

The u-PA obtained by expression of this cDNA is expected to have a similar amino acid sequence to that described in Holmes et al., except for the following alteration:

```
position 6               10   46            50
         -gln-val-pro-ser-asn-lys-ser-lys-thr-cys
```

In a second preferred embodiment, a modified urokinase-type plasminogen activator enzyme is prepared by a process which comprises effecting a deletion mutagenesis upon the cDNA which codes for urokinase-type plasminogen activator using an oligodeoxyribonucleotide comprising a nucleotide sequence which is complementary or corresponds to a region of the u-PA cDNA extending 5′ from nucleotide 205 inclusive, said sequence being joined directly to a nucleotide sequence which is complementary or corresponds to a region extending 3′ from nucleotide 215 inclusive of u-PA cDNA and expressing the modified cDNA in a prokaryote or eurokaryote host.

The oligodeoxyribonucleotide employed in the preferred embodiment is suitably at least 16 nucleotides in length, preferably at least 30 nucleotides in length. Suitably it contains at least 8 and preferably at least 15 nucleotides which are complementary to nucleotides on each side of the deletion region.

A preferred oligodeoxyribonucleotide is of sequence (I).

5′d(GCACCAGTGAATGTTCTTGTTGGACACACA)3′ (I)

The oligodeoxyribonucleotide of sequence (I) is novel and as such forms part of the invention.

The 15 bases at the 5′ end of the oligodeoxyribonucleotide (I) are complementary to u-PA cDNA nucleotides 229-215 inclusive and the 15 bases at the 3′ end of the oligodeoxyribonucleotide (I) are complementary to the u-PA cDNA nucleotides 205-191 inclusive.

The oligonucleotide of sequence (I) can be used to delete the portion of cDNA which codes for amino acids 24 to 26 inclusive of u-PA. It is believed that the cDNA obtained following mutagenesis is similar to that of Holmes, W.E. et al (op. cit.) with the following new nucleotide sequence:-

```
5' - GTG TCC AAC AAG AAC ATT CAC TGG 3'
      |            | |          |
position 194     205 215       226
```

The u-PA obtained by expression of this cDNA is expected to have a similar amino acid sequence to that described in Holmes et al., except for the following alteration:

```
position 20         23  27        30
         -val-ser-asn-lys-asn-ile-his-trp-
```

The modified u-PA of the invention comprises the B-chain of native u-PA linked to u-PA A-chain modified in the region of the growth factor domain, such as in the region of amino acids 24 to 26. This modified u-PA A-chain may be employed as one chain of a fibrinolytically active hybrid protein such as disclosed in EP-0155387. The modified u-PA A-chain, DNA encoding the modified u-PA A-chain and a hybrid protein comprising the modified u-PA A-chain linked to the B-chain of a fibrinolytically active protease, the catalytic site of which is optionally blocked by a removable blocking group, all form part of the invention. The hybrid protein may be used in any of the methods and compositions described hereinafter for the modified u-PA itself.

The modified u-PA of the invention is suitably administered in the form of a pharmaceutical composition.

Accordingly the present invention also provides a pharmaceutical composition comprising modified u-PA of the invention in combination with a pharmaceutically acceptable carrier.

The compositions according to the invention may be formulated in accordance with routine procedures as pharmaceutical compositions adapted for intravenous administration to human beings.

Typically compositions for intravenous administration are solutions of the sterile enzyme in sterile isotonic aqueous buffer. Where necessary the composition may also include a solubilising agent to keep the modified u-PA in solution and a local anaesthetic such as lignocaine to ease pain at the site of injection. Generally, the modified u-PA will be supplied in unit dosage form for example as a dry powder or water free concentrate in a hermetically sealed container such as an ampoule or sachette indicating the quantity of protein in activity units. Where the modified u-PA includes a removable blocking group an indication of the time within which the free protein will be liberated may be given. Where the protein is to be administered by infusion, it will be dispensed with an infusion bottle containing sterile pharmaceutical grade 'Water for Injection' or saline. Where the protein is to be administered by injection, it is dispensed with an ampoule of sterile water for injection or saline. The injectable or infusable composition will be made up by mixing the ingredients prior to administration. The quantity of material administered will depend upon the amount of fibrinolysis required and the speed with which it is required, the seriousness of the thromboembolic condition and position and size of the clot. The precise dose to be employed and mode of administration must per force in view of the nature of the complaint be decided according to the circumstances by the physician supervising treatment. However, in general, a patient being treated for a thrombus will generally receive a daily dose of from 0.01 to 10 mg/kg of body weight either by injection in for example up to five doses or by infusion.

Within the above indicated dosage range, no adverse toxicological effects are indicated with the compounds of the invention.

Accordingly, in a further aspect of the invention there is provided a method of treating thrombotic diseases, which comprises administering to the sufferer an effective non-toxic amount of modified u-PA of the invention.

The invention also provides a modified u-PA of the invention for use as an active therapeutic substance and, in particular, for use in the treatment of thrombotic diseases.

The following Examples illustrate the invention.

## Example 1

5' d(GCAGGTTTTTGACTTGTTCGATGGAACTTG) 3'

The abovementioned 30-mer is prepared by conventional solid-phase methods analogous to those described herein.

The oligodexyribonucleotide can be used to produce modified u-PA cDNA using methods described in Nucl. Acid. Res.; 1984, 12, 2407-2419 or Biochem. Soc. Trans., 1984, 12 , 224-225.

Expression of the modified cDNA in prokaryote or eukaryote host will yield modified u-PA of the invention in which amino acids 11 to 45 inclusive are deleted.

## Example 2

5' d(GCACCAGTGAATGTTCTTGTTGGACACACA)3'

The abovementioned 30-mer is prepared by conventional solid-phase methods analogous to those described herein, and used to prepare modified u-PA of the invention in which amino acids 24 to 26 are deleted, by the procedures of Example 1.

## Claims

1. A fibrinolytically active urokinase-type plasminogen activator (u-PA) which has been modified in the region of the growth factor domain, the catalytic site essential for fibrinolytic activity of which is optionally blocked by a removable blocking group.

2. A modified u-PA according to claim 1, which comprises amino acids 1 to 10 of native u-PA.

3. A modified u-PA according to claim 1 or 2 in which the modification comprises the deletion of residues 11 to 45.

4. A modified u-PA according to claim 1 or 2 in which the modification comprises the deletion of residues 24 to 26 .

5. A hybrid protein comprising urokinase-type plasminogen activator A-chain which has been modified in the region of the growth factor domain, linked to the B-chain of a fibrinolytically active protease, the catalytic site of which is optionally blocked by a removable blocking group.

6. An enzyme-protein conjugate in which the catalytic site on the enzyme which is responsible for fibrinolytic activity is blocked by protein attached thereto by way of a reversible linking group, wherein one of the enzyme and the protein is a modified u-PA according to any of claims 1 to 4.

7. A pharmaceutical composition comprising modified u-PA according to claim 1, in combination with a pharmaceutically acceptable carrier.

8. A modified u-PA according to claim 1 for use in the treatment of thrombotic diseases.

9. Use of a modified u-PA according to claim 1 in the preparation of a medicament for the treatment of thrombotic diseases.

10. A process for preparing a modified u-PA according to claim 1, which process comprises expressing DNA encoding said modified u-PA in a recombinant host cell and recovering the modified u-PA product.

European Patent Office

# EUROPEAN SEARCH REPORT

Application number EP 87307496.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP - A1 - 0 009 879 (BEECHAM GROUP LIMITED)<br>* Abstract; pages 1,2 *<br>-- | 1,7-9 | C 12 N 15/00<br>C 12 N 9/68<br>A 61 K 37/54 |
| P,A | EP - A1 - 0 207 589 (BEECHAM GROUP PLC)<br>* Claims 1,12-15,21 *<br>-- | 1,5,7-10 | |
| D,A | NUCLEIC ACIDS RESEARCH, vol. 13, no. 8, April 25, 1985 (Oxford,GB)<br>A. RICCIO et al. "The human urokinase-plasminogen activator gene and its promoter" pages 2759-2771<br>* Totality *<br>-- | 1,10 | |
| D,A | BIOTECHNOLOGY, vol. 3, October 1985 (Martius ville, USA)<br>W.E. HOLMES et al. "Cloning and Expression of the Gene for Pro-urokinase in Escherichia coli" pages 923-929<br>* Totality *<br>-- | 1,10 | TECHNICAL FIELDS SEARCHED (Int. Cl.4)<br>C 12 N<br>A 61 K |
| D,A | FEBS LETTERS, vol. 163, no. 1, October 1983 (Amsterdam)<br>L. BANYAI et al. "Common evolutionary origin of the fibrin-binding structures of fibronectin and tissue-type plasminogen activator" pages 37-41<br>* Totality *<br>-- | 1 | |

The present search report has been drawn up for all claims

| Place of search | Date of completion of the search | Examiner |
|---|---|---|
| VIENNA | 09-12-1987 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

EP 87307496.7

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| D,A | BLOOD, vol. 67, no. 6, June 1986 (New York)<br>M. VERSTRAETE et. al. "Thrombolytic Therapy in the Eighties" pages 1529-1541<br>* Totality *<br>---- | | |
| | | | TECHNICAL FIELDS SEARCHED (Int. Cl.4) |

The present search report has been drawn up for all claims

| Place of search | Date of completion of the search | Examiner |
|---|---|---|
| VIENNA | 09-12-1987 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82